(19) **Europäisches Patentamt**
**European Patent Office**
**Office européen des brevets**

(11) **EP 1 408 830 B1**

(12) **EUROPEAN PATENT SPECIFICATION**

(45) Date of publication and mention
of the grant of the patent:
**14.03.2007 Bulletin 2007/11**

(51) Int Cl.:
*A61B 5/05* *(2006.01)*     *A61H 39/00* *(2006.01)*

(21) Application number: **00954900.7**

(22) Date of filing: **16.08.2000**

(86) International application number:
**PCT/IT2000/000341**

(87) International publication number:
**WO 2001/012062 (22.02.2001 Gazette 2001/08)**

(54) **INSTRUMENT AND METHOD OF REFLEXOLOGICAL MEASUREMENT TO STANDARDIZE AND CLASSIFY A NEW MEASURE UNIT**

INSTRUMENT UND VERFAHREN UM EINE NEUE MESSEINHEIT ZU STANDARDISIEREN UND KLASSIFIZIEREN

APPAREIL ET PROCEDE DE MESURE REFLEXOLOGIQUE SERVANT A NORMALISER ET CLASSIFIER UNE NOUVELLE UNITE DE MESURE

(84) Designated Contracting States:
**AT BE CH CY DE DK ES FI FR GB GR IE IT LI LU MC NL PT SE**

(30) Priority: **17.08.1999 IT CS990012**

(43) Date of publication of application:
**21.04.2004 Bulletin 2004/17**

(73) Proprietors:
• **Tecnap S.r.l.**
**87036 Rende/CS (IT)**
• **Meta Instrument SRL**
**87036 Rende (IT)**

(72) Inventors:
• **FERRARO, Vincenzo**
**I-87036 Rende (IT)**
• **TKACHENKO, Yuri**
**Niznj Novgorod, 603600 (RU)**

(56) References cited:
**CH-A- 618 863**          **DE-A- 2 552 190**
**DE-A- 19 717 337**       **FR-A- 2 608 917**

• **ISHCHENKO A N ET AL: "AURICULAR DIAGNOSTICS USED IN THE SYSTEM OF SCREENING SURVEYS" MEDICAL PROGRESS THROUGH TECHNOLOGY,DE,SPRINGER VERLAG. BERLIN, vol. 17, no. 1, 1991, pages 29-32, XP000235807 ISSN: 0047-6552**
• **IONESCY-TIRGOVISTE C ET AL: "ELECTRIC DIAGNOSIS IN ACUPUNCTURE" AMERICAN JOURNAL OF ACUPUNCTURE,US, ACUPUNCTURE RESEARCH PUBL. CO., FELTON, CA, vol. 12, no. 3, 1 July 1984 (1984-07-01), pages 229-238, XP000578641 ISSN: 0091-3960**

**Description**

FIELD OF THE INVENTION

The instrument and the method of reflexological measurement object of the present invention standardizes and classifies a new measure unity for the electromedical sector, suitable to signal, by means of a proper measure/ elaboration of electrical conductivity in some points of the auricular pavilion, the diagnose of some pathologies.

STATE OF THE ART AND BETTER WAY TO CARRY OUT THE INVENTION

The invention has the purpose to realize:

✓ **STANDARDIZATION OF THE MEASURE ACQUISITION PROBE;;**
✓ **ADAPTATION/CALIBRATION OF THE MEASURES, USING A SUITABLE HARDWARE STRUCTURE, IN ORDER TO BE RELATED TO THE SPECIFIC CHARACTERISTICS OF THE PATIENT;**
✓ **ELABORATION/TRANSDUCTION OF THE MEASURES IN ORDER TO OBTAIN A PARAMETER INDICATING THE DIFFERENT PATHOLOGICAL CONDITIONS AND NOT DEPENDING FROM THE SAME REFLEXOLOGICAL SENSITIVITY OF THE ANALYZED ORGANS..**

Hereinafter the innovative methods are illustrated in the single sections in order to obtain this new measure standard that will be called FTDB (Ferraro/Tkachenko Data-Base).

**STANDARDIZATION OF THE MEASURE ACQUISITION PROBE**

Many scientific publications describe how the electrical resistance of the human body is modified in the reflexological points, for the reason that it has never been activated a standardization process of the measure, the method has always appeared "not usable/exportable beyond the experience of the single physician, exactly because it is unable to express, in a general way, a parameter that can be repeated and compared; it is in fact known that the electrical resistance on the skin (therefore also on the reflexological points) is modified with the variation of the contact surface between measure organ and epidermic surface changes, or with variation of the pressure by which the measure instrument presses to realize the contact on the skin.
Our standardization process starts therefore with the structuring of the probe (section that surveys/transduces the value of the electrical resistance of the human body), carrying out to this purpose a series of clinical tests/comparisons in order to obtain the best functional parameters; that is very complex because:

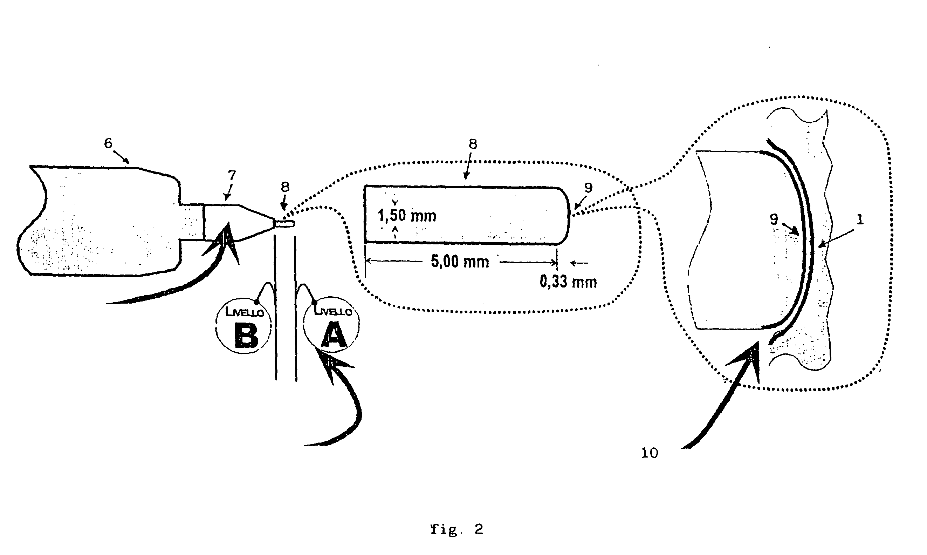

(a) contact surface should be as wide as possible, without that for this reason the measure is affected/mediated by the resistive value of measures other then the auricular diagnostic point under examination;
(b) contact surface must be curved, because it must be able to assure a good uniform and repetitive contact between skin and measure probe, without creating to the patient pain conditions (ear is a measure area particularly sensible);
(c) contact pressure between measure element and reflexological point must be able to assure a good contact between the parts, but in the mean time it must be the less elevate as possible; it could so far squeeze/damage the skin in the contact point and therefore could alter the measure.
At the end of the experimental phase the best parameters were the ones quoted in figure 2. Consequently as standard for FTDB measure unity has been defined a probe that should have the following characteristics:
(d) the point of measure, from now on denominated PTM, must be realized with a conductive material (in our experimental application it has been built with stainless steel) and must have a diameter of 1,5 mm;
(e) PTM, normally positioned on level-A (see fig.2) must be able to draw back till to reach level-B and in this position it must assure a pressure of 65 grams between PTM and skin (our prototype has been realized with a phosphorous bronze spring that can be suitably calibrated by means of a screw system); user finds very easy to reach level-B because this level coincides with complete drawback of PTM and therefore with the contact on the skin of the insulated material part (see insulated material part fig.2);
(f) Contact surface that PTM offers to the skin must have a "uniformly lowered spherical" shape whose maximum diameter is equal to 1.5 mm, while the minimum diameter is 0.66 mm; a good compromise that clinical research activated to this purpose has showed able to satisfy the different exigencies that, in this case, are to be mediated: repetitive contact surface in different reflexological points, absence of pain/trouble on the patient. The experimental prototype has been realized by means of an electroerosion process, in order to operate in absolute safety as regards to the qualitative/dimensional aspects of the finished product.

User, positioning PTM to perform the measure, shall be obviously very careful to position the probe in a perpendicular way to the natural proceeding of the skin on the reflexological point that is to be analyzed; in fact only in this way it is sure that conduction surface (and therefore the value of the measure) is in conformity to the one foreseen in the clinical experimentation phase and that, consequently, the observed data shall report, exactly diagnostic results that reflexological process makes evident on a patient that undergoes to examination.

**ADAPTATION/CALIBRATION OF THE MEASURE, USING A SUITABLE HARDWARE STRUCTURE, IN ORDER TO BE RELATED TO THE SPECIFIC CHARACTERISTICS OF THE PATIENT.**

Saying first that this description uses two terms nominated point-zero (from now on indicated as PNTZ) and reflexological point of the organ under examination (from now on indicated as PROE), which corresponds to the point AT-83 and to any one of the diagnostic point of measure of the standard Konig-Wancura, it is made evident that the eventual pathological datum that auricular diagnostic system points out it is contained just in the difference of measure (unbalance) existing between the point PNTZ and the point PROE (in some cases the pathological condition is evaluated in an indirect way, that is correlating other reflexological points, without modifying the functional concept of the method.

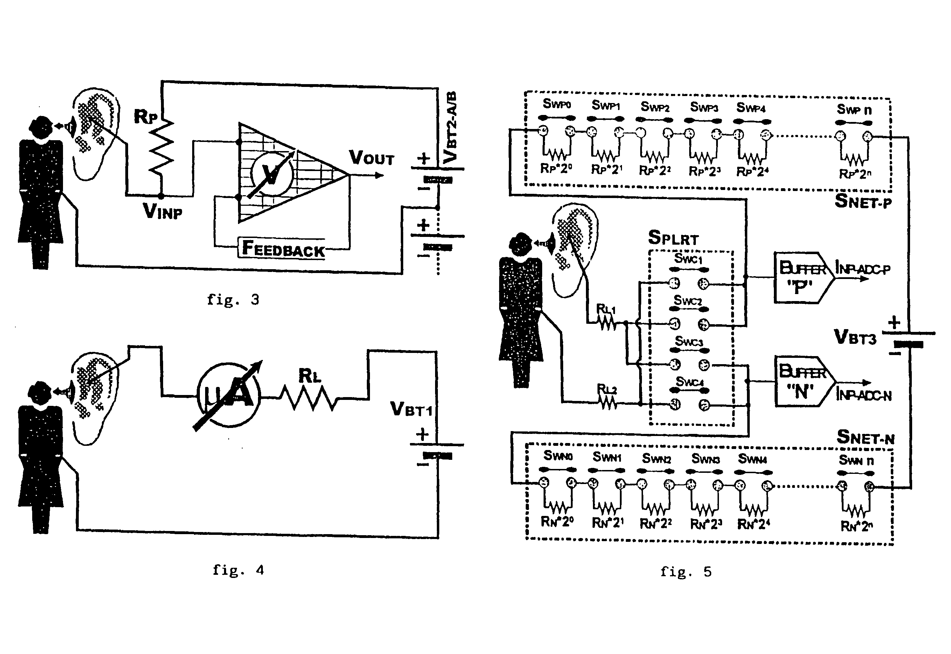

To find out these measures actually transduction standards that utilizes a "microAmperometer" system (see fig.3) or a "resistive partition" system (see fig.4), two methodologies of measure that, as we will demonstrate, are little efficacious in this typology of application and undoubtedly, given their low reliability/repetitivity they have considerably slowed down the affirmation of the auricular diagnostic method as "powerful method of diagnosis.

In the case of measurement by means of **"MICRO-AMPEROMETER"**, in fact, the resistive difference that shows the pathological condition level, is got in a strongly approximate way and with a low level of repetitivity/comparison between patient and patient, in fact, if two patient, that are put in comparison, have different ohmic resistance in point PNTZ, no serious comparison is possible, really the system does not allow reliable comparisons, in different times, not even on the same patient. It has been experimented that PNTZ of the same individuals changes if hours and/or days change.

To demonstrate the problems that, in such a use, this typology brings, the following association labels/measure parameter are assigned (making reference to fig. 3).

&check; **"$R_{PTG}$"** indicates the resistance that expresses the pathological condition (difference between the resistance of PNTZ point and the one of PROE point);
&check; **"$I_{PNTZ}$"** indicates the current that is present on the point PNTZ;
&check; **"$R_{PNTZ}$"** indicates the resistance that is present on the point PNTZ;
&check; **"$I_{PROE}$"** indicates the current that is present on the point PROE;
&check; **"$R_{PROE}$"** indicates the resistance that is present on the point PROE;
&check; **"$V_{BT1}$"** indicates the tension that is present on the feeding knot;
&check; **"$R_L$"** indicates the resistance that is assigned as current limiter;

and from these parameters it is possible to get the equation that elaborates the resistive value on PNTZ, that is expressed as it follows :

$$Rpntz = \frac{Vbt1 - (Ipntz * Rl)}{Iptnz}$$

the equation that gets the resistive value on point PROE, that is expressed as it follows:

$$Rproe = \frac{Vbt1 - (Iproe * Rl)}{Iproe}$$

To obtain the value that expresses the parameter of the pathological condition:

$$Rptg = Rpntz - Rproe$$

this is a parameter that is interpreted in an "absolute way" and therefore is not correlated to the whole of the dynamical condition that characterizes the same measure, said parameter becomes not significative as the value of **"R$_{PNTZ}$"**, is modified, this is a heavy limitation to the system that becomes not usable for the formation of data bases and/or of interpretative "objective" standards.

In the case of the measure by means of **"RESISTIVE-DIVIDER"** (see fig. 4), besides the problems of objective safety for the patient, the method is directed, in fact, to the use of operational amplifier (whose functional standards of tension are rather elevated, typically comprised between +/- 10 and +/- 15 Volts;

- for a potential difference of tension, if something is wrong, comprised between 20 and 30 Volts, the system presents an intrinsic functional non linear conception of its and for this reason it presents a "comparative deficiency" of the provided measures.

If we want to demonstrate, in this schematic typology the errors that characterize the system we assign, as for preceding case, the following association of labels/parameters of measure:

- **"V$_{BT2A/B}$"** indicates the feeding tension (single or dual) on the feeding knot;
- **"V$_{PNTZ}$"** indicates the tension of point VINP when it is measured PNTZ;
- **"V$_{PROE}$"** indicates the tension of point VINP when it is measured VPROE;

 ✓ **"V$_{PTG}$"** indicates the difference between tension V$_{PNTZ}$ and V$_{PROE}$;

- **"R$_P$"** indicates the divider resistance used;

Supposing, in this case, that we want to actuate a comparative diagnosis between two patients that are indicated with suffix A and B , which present respectively a R$_{PNTZ}$-A of 150 KΩ, and a R$_{PNTZ}$-B of 350 KΩ, on a R$_P$ of 250 KΩ, , in this case we should have in the case "A" a V$_{INP}$ of:

$$VpntzA = \frac{Vbt2AB}{Rp + RpntzA} * RpntzA = \frac{Vbt2AB * 150.000}{250000 + 150000} = 0,375 * Vbt2AB$$

and in the case B a V$_{INP}$ of:

$$VpntzB = \frac{Vbt2AB}{Rp + RpntzB} * RpntzB = \frac{Vbt2AB * 350.000}{250000 + 350000} = 0,583 * Vbt2AB$$

considering, as demonstrative example, a variation of R$_{PROE}$ in positive sense and in both cases of 35%, we would register in the case A a V$_{PROE}$ of:

$$VproeA = \frac{Vbt2AB}{Rp + RproeA} * RproeA = \frac{Vbt2AB * 202.500}{250000 + 202.500} = 0,447 * Vbt2AB$$

and in the case B a V$_{PROEB}$ of:

$$VproeB = \frac{Vbt2AB}{Rp + RproeB} * RproeB = \frac{Vbt2AB * 472.500}{250000 + 472.500} = 0,654 * Vbt2AB$$

an error of 46% while the two parameters should be perfectly equal because they are generated from the same quantity of initial delta: +35%.

The circuital solution of the present invention can be called: **"VARIABLE RESISTIVE DIVIDERS NET"** (see fig.5), it allows remarkable functional advantages, between these ones:

✔ an operative tension of absolute safety (it does not require, in spite of the elevated precision, that allows, the use of operational amplifiers);
✔ survey of measures structurally without errors;
✔ possibility of balancing any effect of electrolysis;
✔ possibility of being disposed as easy programmable system of therapeutic rebalancing;

Hereinafter we describe the characteristics/performances of the circuit, together with the list of the advantages that its structuring allows.

To characterize its block scheme, two divider nets are used, , (indicated as Snet-p Snet-n in the scheme), they are constituted by a series of analogical switches in C-Mos connected in parallel with a series of calibrated resistances with a binary scale (in the construction of the prototype the value of 5 K$\Omega$ has been assigned to Rp).

This divider, enabling opportunely the control of the analogical switches ($S_{WP0}$, $S_{WP1}$ ... $S_{WPn}$) may acquire, with a step of 5 K$\Omega$, any resistive value comprised between 0 K$\Omega$ (activating all the controls of the analogical switches) and the sum of all the resistances connected on the resistive net (putting of all the controls of the analogical switches); in our prototype, in which are inserted 8 analogical switches, the device could therefore operate giving to the circuit a divider resistance programmable between 0 K$\Omega$ and 1.275 M$\Omega$.

A rather important consideration is that both the reading precision and the extension of the maximum resistance, both may be calibrated to the parameter considered the most important, modifying the number of switches and/or the value of the base resistance $R_P$.

It is important to make evident that the precision of reading of our prototype (8 analogical switch on a base resistance $R_P$ of 5 K$\Omega$), is not to be intended as a structure that has a limit precision of 5 K$\Omega$, in fact, if we assign, as in the preceding cases, the labels/parameters associations of measure:

✔ **"$R_{EAR}$"** indicates the resistance on the auricular diagnostic point that is intended to measure;
✔ **"$R_{NET-P}$"** indicates the resistance on the auricular diagnostic point that is supplied, on the analogical switches, by means of programming, to the net of the positive divider;
✔ **$V_{BT3}$"** indicates the feeding tension of the circuit;
✔ **"$V_{ADC}$"** indicates the measure tension on the variable resistive divider (measured by the analogical/digital converter and equivalent to $V_{BT}3/256$ for every digit);
✔ **"$R_{PRV}$"** indicates the programmed resistance on the variable-resistive-divider;

and considering, for example, an operative condition in which:

✔ the net of the negative analogical switches **($S_{NET-N}$)**, is programmed in such a way that can be totally activated and therefore putting this section with a value of the resistance zero;
✔ the net of the polarity switches **($S_{PLRT}$)**, is programmed in such a way that only the analogical switches $S_{WC2}$ e $S_{WC4}$ are active; in order to make the measure net to became operative as a normal divider positioned on the positive pole;

An operative logic is actuated, in which, in a first phase, the control of the analogical switches $S_{NET-P}$ searches for a $R_{NET-P}$, that is as near as possible to the $R_{EAR}$.

(This condition is reached searching for the value of $V_{ADC}$ that is as near as possible to "#128" - if we operate with a net of 8bits) and optimizing the reached result, activating the equation that gets the effective value of $R_{EAR}$, through the under reported formula:

$$\mathrm{Re}\,ar = \frac{Vadc}{Iprv} = \frac{Vadc}{\dfrac{Vbt3 - Vadc}{Rprv}}$$

this elaboration allows to get the $R_{EAR}$ value with an approximation even more detailed than the one obtainable by means of only a net of resistive divider $R_{NET-P}$.

The formula that from a first analysis seems strongly approximate, it can be really considered valid because: the values $R_{L1}$ and $R_{L2}$ are negligible in comparison with the playing quantity, the Buffer-P presents an incoming impedance that, also in this case, is negligible in comparison with the operational parameters as the impedance of short circuits and/or open circuit of analogical switches are.

Regarding to the functional aspects of the system and evaluating the same under a potential/total profile, it is, at the end, made evident, that:

1. the analogical switches of polarity **"S$_{PLRT}$"**, activating opportunely their control, allow:

a) to close electrically the reflexological point under examination (in our prototype activating controls: $S_{WC1}$, $S_{WC2}$, $S_{WC3}$, $S_{WC4}$);
b) to open electrically the reflexological point under examination (in our prototype inactivating the controls: $S_{WC1}$, $S_{WC2}$, $S_{WC3}$, $S_{WC4}$);
c) to polarize in a positive way the reflexological point under examination (in our prototype activating controls: $S_{WC2}$, $S_{WC4}$);
d) to polarize in a negative way the reflexological point under examination (in our prototype activating controls: $S_{WC1}$, $S_{WC3}$); and from these possible functional conditions, it is then possible to arrange to activate anti electrolytic, of equalization, therapeutical correction.

2. The analogical switches of the resistive divider positioned on the positive polarity **"S$_{NET-P}$"**, activating opportunely their controls (selectable from $S_{WP0}$ till $S_{WP7}$ - as said our prototype has been structured at 8 bits, but could also be at 16 or 32 bits) allow:

e) To position themselves on different levels of the positive electric potential and therefore to generate specific/particular forms of waves able to compensate in the most suitable way the most right actions necessary to the reflexological points under examination.

3. The analogical switches of the resistive divider, positioned on the negative polarity **"S$_{NET-N}$"**, activating opportunely their controls (selectable from $S_{WN0}$ till $S_{WN7}$- as said our prototype has been structured at 8 bits, but could also be at 16 or 32 bits) allow:

f) To position themselves on different levels of the negative electric potential and therefore to generate specific/particular forms of waves able to compensate in the most suitable way the most right actions necessary to the reflexological points under examination.

The instrument of the present invention allow a series of advantages as:

I. **BETTER OPERATIVE SAFETY =** it can function with a great precision and without the use of operational amplifier and/or high operative tensions; it is adapt to the C-Mos digital standards: +3,3 or +5 Volt d.c.
II. **PERFORMING MEASURES WITH COMPARATIVE TECHNIC =** it can perform a comparison between parameters surveyed with the " active divider" positioned both on the positive polarity and on the negative one; All this to the advantage of the quality/reliability of the measure, because it is possible to actuate a double comparison.
III. **SUPPLYING MEASURE PARAMETERS WITHOUT ERRORS DUE TO NON LINEARITY OF THE TRANSDUCTION** = it can perform the measurement, both trying in an automatic way the same value of $R_{EAR}$ (and using the reading $I_{NP-ADC-P/N}$ as level of control to reach the partition $V_{BT3/2}$), or actuating other forms of partition/reading, in which it is always operative the possibility to change the resistance of partition both on the positive polarity and on the negative one;
IV. **REBALANCING/PRODUCTION OF THERAPEUTICAL EFFECTS** = it can perform compensative reflexological forms modifying:

✔ Polarities of the current/tension (through analogical switches of $S_{PLRT}$);
✔ impedance of the reflexological point (changing opportunely analogical switches of $S_{PLRT}$);
✔ The "wave forms" generating different levels of electric potential (through analogical switches of $S_{NET-P}$ and of $S_{NET-N}$).

## ELABORATION/TRANSDUCTION OF THE MEASURES TO OBTAIN A PARAMETER INDICATOR OF THE DIFFERENT PATHOLOGICAL CONDITIONS, INDEPENDENT FROM THE SAME REFLEXOLOGICAL SENSIBILITY OF THE ANALYZED ORGANS.

Actual methods of auricular diagnostic control, utilizing the value of the delta of measure of any single organ (regarding to zero point) and, to perform the diagnosis, they compare every delta with the different references of pathological state (values are always specific and directed to any particular organ interested); a true confusion of microAmper and/or KΩ where is very difficult to understand.

This new invention moves different reflexological data in a new standard/unity of measure, called FTDB (Ferraro/Tkachenko Data-Base) whose undoubted advantage is that to supply as expression of the diagnostic datum, a value that, independently from the analyzed organ, gives immediately the pathological state that becomes evident on the patient under examination, through its reflexological analysis.

To make even more easy its interpretation, this unity of measure has been structured/divided in four operative areas so classified:

(g) **AREA WITH NON PATHOLOGICAL CONDITIONS:** where values of the FTDB unity are comprised between 0 and 24 and with polarity both positive and negative;
(h) **AREA WITH LIGHT PATHOLOGICAL CONDITIONS:** where values of the FTDB unity are comprised between 25 and 49 with polarity both positive and negative;
(i) **AREA WITH MEDIUM PATHOLOGICAL CONDITIONS:** where values of the FTDB unity are comprised between 50 and 74 and with polarity both positive and negative;
(j) **AREA WITH STRONG PATHOLOGICAL CONDITIONS:** : where values of the FTDB unity are comprised between 75 and 99 and with polarity negative;

a process of standardization that has required to structure a series of curves/matrixes-FTDB of transduction which for every specific organ to evaluate are, in the phase of programming, loaded in the computer to be later on recalled when it is required to elaborate/express the diagnosis as FTDB unity.

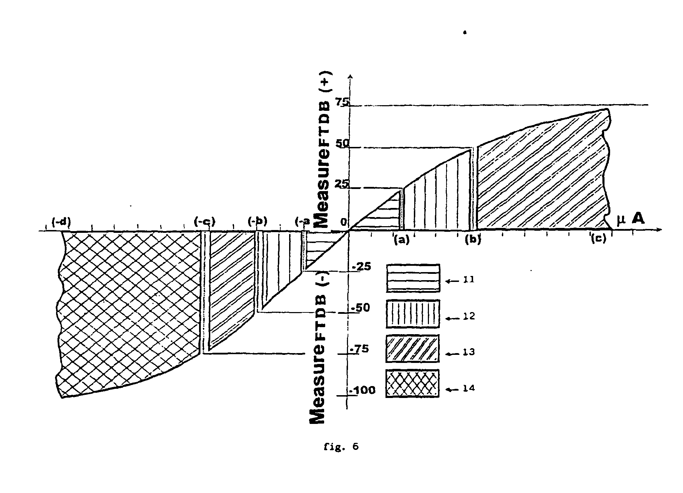

Furthermore, because the different reflexological points are not indicators of the same pathological gravity and/or of a constant direct interpretation (some people gives pre informations to be correlated with other points). Three different types of curves/matrixes classified in this way have been structured: Curves that expresses areas even with "SERIOUS PATHOLOGICAL CONDITIONS" whose example is given in fig. 6 where the definition parameters have been elaborated for the organs and with the delimitations hereinafter reported (curves are referred to theoretic patients whose zero resistance was re-elaborated at 250KΩ):

1. <u>STOMACH</u> (point AT-87) whose principal points of definition are: **"a"** (equivalent to +25 FTDB) with +2,2 μA; **"-a"** (equivalent to -25 FTDB) with -2,0 μA; **"-c"** (equivalent to -75 FTDB) with -6,15 μA.
2. <u>LARGE INTESTINE</u> (point AT-91) whose principal points of definition are: **"a"** (equivalent to +25 FTDB) with +1,6 μA; **"-a"** (equivalent to -25 FTDB) with -1,3 μA; **"-c"** (equivalent to -75 FTDB) with -3,15 μA.
3. <u>PANCREAS</u> (point AT-96) whose principal points of definition are: **"a"** (equivalent to +25 FTDB) with +1,2 μA; **"-a"** (equivalent to -25 FTDB) with -1,0 μA; **"-b"** (equivalent to -50 FTDB) with -2,8 μA.
4. <u>PROSTATE</u> (point AT-93) whose principal points of definition are: **"a"** (equivalent to +25 FTDB) with +1,8 μA; **"-a"** (equivalent to -25 FTDB) with -1,5 μA; **"-c"** (equivalent to -75 FTDB) with -3,8 μA.
5. <u>UTERUS CERVIX</u> (point AT-56) whose principal points of definition are: **"a"** (equivalent to +25 FTDB) with +1,7 μA; **"-a"** (equivalent to -25 FTDB) con -1,5 μA; **"-c"** (equivalent to -75 FTDB) with -3,5 μA.
6. <u>UTERUS</u> (point AT-58) whose principal points of definition are: **"a"** (equivalent to +25 FTDB) with +1,8 μA; **"-a"** (equivalent to -25 FTDB) with -1,5 μA; **"-c"** (equivalent to -75 FTDB) con 3,8 μA.

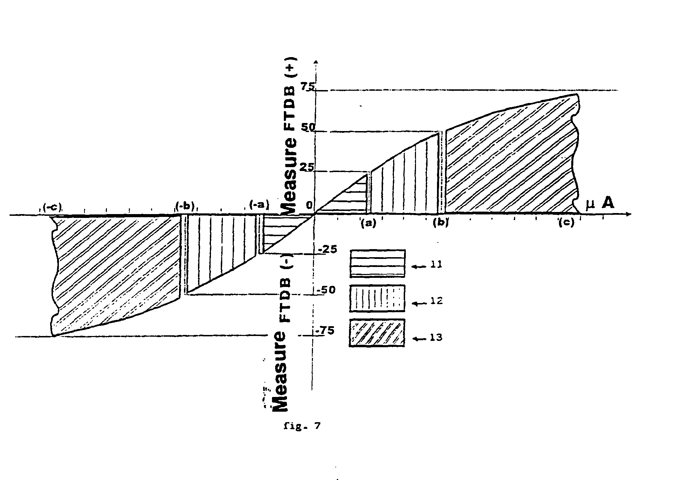

(k) Curves that expresses areas even with "MEDIUM PATHOLOGICAL CONDITIONS", whose example is given in fig. 7 and where the definition parameters have been elaborated for the organs and with the delimitations hereinafter reported (curves are referred to theoretic patients whose zero resistance was re-elaborated at 250KΩ):

1. <u>DUODENUM</u> (point AT-88) whose principal points of definition are: **"a"** (equivant to +25 FTDB) with +1,2 μA; **"-a"** (equivalent to -25 FTDB) with -1,4 μA.

2. <u>BLADDER</u> (point AT-92) whose principal points of definition are: **"a"** (equivalent to +25 FTDB) with +2,5 $\mu$A; **"-a"** (equivalent to -25 FTDB) with -2,5 $\mu$A.

3. <u>URETHRA</u> (point AT-94) whose principal points of definition are: **"a"** (equivalent to +25 FTDB) with +2,0 $\mu$A; **"-a"** (equivalent to -25 FTDB) with -2,0 $\mu$A.

4. <u>KIDNEY</u> (point AT-95) whose principal points of definition are: **"a"** (equivalent to +25 FTDB) with +2,5 $\mu$A; **"-a"** (equivalent to -25 FTDB) with -2,3 $\mu$A.

5. <u>LIVER</u> (point AT-97) whose principal points of definition are: **"a"** (equivalent to +25 FTDB) with +2,8 $\mu$A; **"-a"** (equivalent to -25 FTDB) with -3,1 $\mu$A.

6. <u>SPLEEN</u> (point AT-98) whose principal points of definition are: **"a"** (equivalent to +25 FTDB) with +1,5 $\mu$A; **"-a"** (equivalent to -25 FTDB) with -1,0 $\mu$A; ; **"-b"** (equivalent to -50 FTDB) with -3,0 $\mu$A,

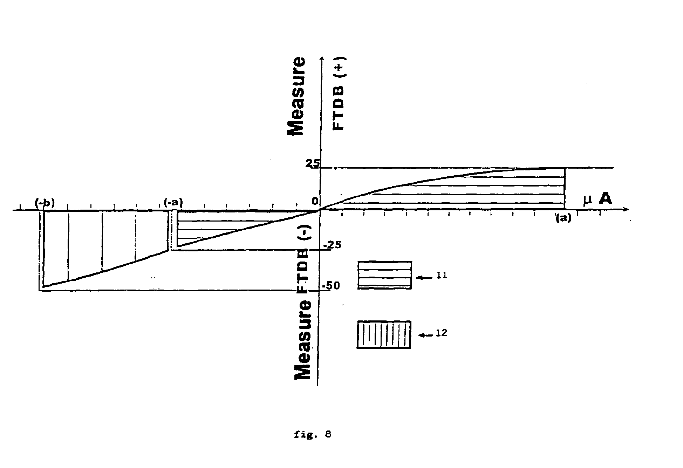

(I) Curves that expresses areas with **"LIGHT PATHOLOGICAL CONDITIONS AND/OR TO REFER TO OTHER PARAMETERS",** "MEDIUM PATHOLOGICAL CONDITIONS", whose example is given in fig. 8 and where the definition parameters have been elaborated for the organs and with the delimitations hereinafter reported (curves are referred to theoretic patients whose zero resistance was re-elaborated at 250K$\Omega$):

1. <u>URETER</u> (point AT-80) whose principal points of definition are: **"-a"** (equivalent to -25 FTDB) with +4,25 $\mu$A;

SYNTHETIC DESCRIPTION OF FIGURES

Other characteristics and advantages of the invention will become clear from the description hereinafter of a way to carry out the invention given as non limitative example and represented from the following figures.

Figura 1 -   scheme of the reflexological instrument of measure.
Figura 2 -   measure probe - magnification of the contact point - magnification of the contact between probe and auricular pavilion.
Figura 3 -   instrument with measure of diagnostic parameters by means of Micro Amperometer.
Figura 4 -   instrument with measure of diagnostic parameters by means of Resistive Divider.
Figura 5 -   instrument with measure of diagnostic parameters by means of Variable Resistive Divider Net.
Figura 6 -   Conversion Table "$\mu$A-FTDB" for measures that put in evidence "Strong pathologies"
Figura 7 -   Conversion Table "$\mu$A-FTDB" for measures that put in evidence "Medium pathologies"
Figura 8 -   Conversion Table "$\mu$A-FTDB" for measures that put in evidence pathological situations to refer to other reflexological points before to define validity/seriousness of measures.

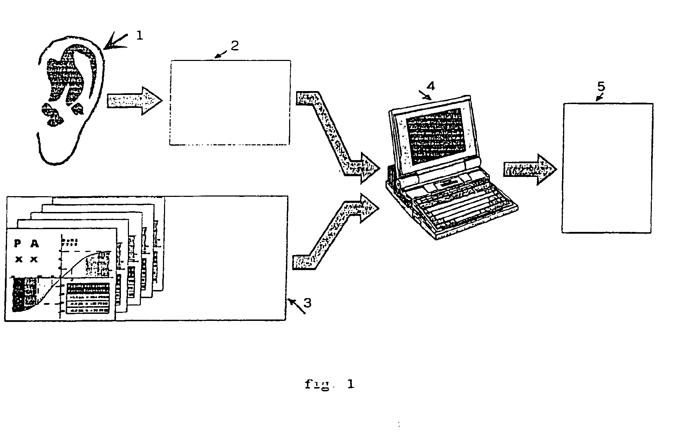

Referring to fig. 1 with (1) is indicated the ear which undergoes to auricular diagnostic, with (2) transductor: Auricular-point/Electric-Resistance, with (3) Matrix for the conversion in FTDB of all the potentially possible values, with (4) a computer with Body-Scanning program, with (5) the diagnostic result expressed in FTDB.

Fig. 2 represents a measure probe (6) that with an insulated block (7) carries a contact point (8) that can be moved between a level A (all out) and a level B (all in) in order to realize a calibrated pressure from "A" to "B" of 65 gr. - the magnification of the contact point (8) shows that the contact surface is rounded - the magnification of the contact between contact point (8) and auricular pavilion (9) shows a surface of contact (10) of 1,95 mm$^2$.

In fig. 6 is represented the conversion table "$\mu$A-FTDB" for measures that put in evidence "strong pathologies", where with (11) areas without pathological conditions are indicated; with (12)) areas with light pathological conditions are indicated; with (13)) areas with medium pathological conditions are indicated; with (14)) areas with strong pathological conditions are indicated.

The value of "$\mu$A " of the Data- base has been elaborated translating experimental measurement, made on the patients under examination, as they were made on "theoretical patients" whose zero-resistance had a constant value of 250 K$\Omega$ (the elaboration is necessary to uniform the measure standard of FTDB).

The limit values (c) and (- d) are obtained using a value double regarding to the maximum experimental measure that has been got in that point; measures that put in evidence even greater lack of balance are put on the same value of the curve FTDB, having already indicated a serious pathological condition.

In fig. 7 is represented the conversion table "$\mu$A-FTDB" for measures that put in evidence "medium pathologies", where with (11) areas without pathological conditions are indicated; with (12)) areas with light pathological conditions are indicated; with (13)) areas with medium pathological conditions are indicated.

The value of "$\mu$A" of the Data- base has been elaborated translating experimental measurement, made on the patients under examination, as they were made on "theoretical patients" whose zero-resistance had a constant value of 250 K$\Omega$ (the elaboration is necessary to uniform the measure standard of FTDB).

The limit values (c) and (- c) are obtained using a value double regarding to the maximum experimental measure that has been got in that point; measures that put in evidence even greater lack of balance are put on the same value of the curve FTDB, having already indicated the maximum pathological condition that the reflexological point under examination allows.

In fig. 8 is represented the conversion table "μA-FTDB" for measures that put in evidence patholgical situations to be referred with other reflexological points before it is possible to define the validity/seriousness, where with (11) areas without pathological conditions are indicated; with (12) areas with light pathological conditions are indicated.

The value of "μA " of the Data- base has been elaborated translating experimental measurement, made on the patients under examination, as they were made on "theoretical patients" whose zero-resistance had a constant value of 250 KΩ (the elaboration is necessary to uniform the meausre standard of FTDB).

The limit values (a) and (- b) are obtained using a value double regarding to the maximum experimental measure that has been got in that point; measures that put in evidence even greater lack of balance are put on the same value of the curve FTDB, having already indicated a pathological condition to be defined with a reflexological correlation.

The present invention of course is not limited to the representation given by the figures, but it may receive improvements and modifications by the man skilled in the art, without going out from the frame of the invention.

The present invention allows numerous advantages and to overcome difficulties that could not be won with systems actually in commerce.

**Claims**

1. Instrument of measurement of the value of the electrical resistance of the human body in the reflexological points made by a measure probe (6), by a circuit by a computer (4) **characterized by** the fact that the circuit has a feeding tension ($V_{BT3}$), that two nets are used, ($S_{NET-P}$, $S_{NET-N}$) one on the positive polarity and another on the negative polarity of the feeding tension that each net is constituted by a series of analogical switches ($S_{WPO}$-$S_{WPN}$, $S_{WNO}$-$S_{WNN}$), connected in parallel with a series of calibrated resistances ($R_{P*2i}$, $R_{N*2i}$) that analogical switches ($S_{WC1}$-$S_{WC4}$) of polarity are present in the circuit, that two resistances ($R_{L1}$, $R_{L2}$) as current limiter are put in the circuit, that the series of analogical switches are controlled in order to obtain a value of the resistance of the series of the calibrated resistances that is near to the value of the point of the ear under measurement, that a data base of measurement which has been elaborated translating experimented measures as if they were made on theoretic patients whose point zero resistance was set 250kΩ, is memorized in the computer, that the computer re-elaborates measurement referring to theoretic patients whose point zero resistance is 250kΩ.

2. Instrument of measurement of resistive value of the skin in the reflexological points for the electromedical sector according to claim 1 **characterized by** the fact that the contact point (8) of the measure probe (6) is retractile from a position all out to a position all in and assures a pressure of 65 gr between contact point (8) and skin (1).

3. instrument of measurement of resistive value on the skin in the reflexological points for the electromedical sector according to claim 2 **characterized by** the fact that the contact point (8) offers to the skin (1) a contact surface (10) with a spherical shape uniformly lowered whose maximum diameter is 1,5 mm while the minimum diameter is 0,66 mm.

4. Instrument of measurement of resistive value on the skin in the reflexological points for the electromedical sector according to claim 2 or 3 **characterized by** the fact that the contact point (8) is made with a conductive material.

5. Method for the reflexological measure that uses an instrument of measurement of resistive value on the skin in the reflexological points for the electromedical sector according to anyone of the preceding claims **characterized by** the fact that

   ➢ the net of the analogical switches put on the negative polarity, is programmed in such a way that can be totally activated and therefore putting this section with a value of the resistance equal to the value of the resistance of point zero;
   ➢ the net of the analogical polarity switches (SPLRT), is programmed in such a way that only the analogical switches $S_{WC2}$ e $S_{WC4}$ are active; in order to make the measure net to become operative on the positive pole;
   ➢ the net of the analogical switches put on the negative polarity, is programmed in such a way that can be totally activated and therefore putting this section with a value of the resistance equal to the value of the resistance of the point of the ear under examination.

6. Method for the reflexological measure that uses an instrument of measurement of resistive value on the skin in the reflexological points for the electromedical sector according to claim 5 **characterized by** the fact that the analogical switches of polarity (SPLRT):

    a) closes electrically the reflexological point under examination activating controls: $S_{WC1}$, $S_{WC2}$, $S_{WC3}$, $S_{WC4}$;

    b) opens electrically the reflexological point under examination inactivating the controls: $S_{WC1}$, $S_{WC2}$, $S_{WC3}$, $S_{WC4}$;

    c) polarizes in a positive way the reflexological point under examination activating controls: $S_{WC2}$, $S_{WC4}$;

    d) polarizes in a negative way the reflexological point under examination activating controls: $S_{WC1}$, $S_{WC3}$.

## Patentansprüche

1. Meßinstrument zur Bestimmung des Wertes des elektrischen Widerstands des menschlichen Körpers an den reflexologischen Stellen mit einem Meßkopf (6), einem Schaltkreis und einem Computer (4), **dadurch gekennzeichnet, daß** der Schaltkreis eine Speisespannung ($V_{BT3}$) hat, daß zwei Netze ($S_{NET-P}$, $S_{NET-N}$) eingesetzt werden, von denen das eine an die positive Polung und das andere an die negative Polung der Speisespannungsquelle angeschlossen ist, daß jedes Netz aus einer Reihenschaltung analoger Schalter ($S_{WPO}$-$S_{WPN}$, $S_{WNO}$-$S_{WNN}$) besteht, die parallel mit einer Reihe geeichter Widerstände ($R_{P*2i}$, $R_{N*2i}$) verbunden sind, daß analoge Polungs-Schalter ($S_{WC1}$-$S_{WC4}$) in dem Schaltkreis vorgesehen sind, daß zwei Widerstände ($R_{L1}$, $R_{L2}$) als Strombegrenzer in den Schaltkreis eingefügt sind, daß die Reihe der analogen Schalter so gesteuert ist, daß sich ein Widerstandswert der Reihe der geeichten Widerstände ergibt, der nahe dem Wert der zu messenden Stelle am Ohr ist, daß eine Meßdatenbasis in dem Computer eingespeichert ist, die erarbeitet wurde, indem experimentell Meßwerte übertragen wurden, als wären diese an theoretischen Patienten erfaßt worden, deren Widerstands-Nullpunkt auf 250 kΩ eingestellt wurde, und daß der Computer die Messung auswertet, indem er sich auf theoretische Patienten bezieht, deren Widerstands-Nullpunkt bei 250 kΩ liegt.

2. Meßinstrument zur Bestimmung des Widerstandswertes der Haut an den reflexologischen Stellen für den elektromechanischen Bereich nach Anspruch 1, **dadurch gekennzeichnet, daß** der Kontaktpunkt (8) des Meßkopfes (6) zurückziehbar ist von einer vollständig ausgefahrenen in eine vollständig eingefahrene Stellung und eine Druckkraft von 65gr zwischen dem Kontaktpunkt (8) und der Haut (1) aufbringt.

3. Meßinstrumente zur Bestimmung des Widerstandswertes der Haut an den reflexologischen Stellen für den elektromechanischen Bereich nach Anspruch 2, **dadurch gekennzeichnet, daß** der Kontaktpunkt (8) der Haut (1) eine Kontaktfläche (10) bietet, die eine sich gleichmäßig abflachende Kugelform hat, bei der der größte Durchmesser 1,5 mm und der kleinste Durchmesser 0,66 mm beträgt.

4. Meßinstrumente zur Bestimmung des Widerstandswertes der Haut an den reflexologischen Stellen für den elektromechanischen Bereich nach Anspruch 2 oder 3, **dadurch gekennzeichnet, daß** der Kontaktpunkt (8) aus leitendem Material besteht.

5. Verfahren zur reflexologischen Messung unter Verwendung eines Meßinstrumentes für die Bestimmung des Widerstandswertes der Haut an reflexologischen Stellen für den elektromechanischen Bereich nach einem der vorhergehenden Ansprüche, **dadurch gekennzeichnet, daß**

    - das Netz der analogen Schalter, die an der negative Polung anliegen, so programmiert wird, daß es vollständig aktiviert werden kann und damit diesen Abschnitt auf einen Widerstandswert bringt, der gleich dem Widerstands-Nullpunkt ist;

    - das Netz der analogen Polungs-Schalter (SPLRT) so programmiert wird, daß nur die analogen Schalter $S_{WC2}$ und $S_{WC4}$ aktiviert werden, so daß das Messnetz am positiven Pol anliegend wirksam wird;

    - das Netz der am negativen Pol anliegenden analogen Schalter so programmiert wird, daß es vollständig aktiviert werden kann und damit diesen Abschnitt auf einen Widerstandswert bringt, der gleich dem Widerstandswert der zu messenden Stelle am Ohr ist.

6. Verfahren zur reflexologischen Messung unter Verwendung eines Meßinstrumentes für die Bestimmung des Widerstandswertes der Haut an reflexologischen Stellen für den elektromechanischen Bereich nach Anspruch 5, **dadurch gekennzeichnet, daß** die analogen Polungs-Schalter (SPLRT)

    a) die zu messende Reflexstelle elektrisch schließen, wozu die Steuerschalter $S_{WC1}$, $S_{WC2}$, $S_{WC3}$, $S_{WC4}$ aktiviert werden,

b) die zu messende Reflexstelle elektrisch öffnen, wozu die Steuerschalter $S_{WC1}$, $S_{WC2}$, $S_{WC3}$, $S_{WC4}$ deaktiviert werden,

c) die zu messende Reflexstelle positiv polen, wozu die Steuerschalter $S_{WC2}$, $S_{WC4}$ aktiviert werden,

d) die zu messende Reflexstelle negativ polen, wozu die Steuerschalter $S_{WC1}$, $S_{WC3}$ aktiviert werden.

## Revendications

1. Instrument de mesure de la valeur de la résistance électrique du corps humain aux points réflexologiques, constitué d'une sonde de mesure (6), d'un circuit, d'un ordinateur (4), **caractérisé par le fait que** le circuit a une tension d'alimentation (VBT3), que deux réseaux sont utilisés (SNET-P, SNET-N), un sur la polarité positive et l'autre sur la polarité négative de la tension d'alimentation, que chaque réseau est constitué d'une série de commutateurs analogiques ($S_{WPO}$-$S_{WPN}$, $S_{WNO}$-$S_{WNN}$) connectés en parallèle à une série de résistances calibrées ($R_{P*2i}$, $R_{N*2i}$) ; que des commutateurs de polarité analogiques ($S_{WC1}$-$S_{WC4}$) sont présents dans le circuit, que deux résistances ($R_{L1}$,$R_{L2}$) sont placées dans le circuit comme limiteurs de courant, que la série de commutateurs analogiques est contrôlée pour obtenir une valeur de la résistance de la série de résistances calibrées qui soit proche de la valeur du point de l'oreille en cours de mesure, qu'une base de données de mesure qui a été élaborée en convertissant des mesures expérimentales comme si elles avaient été faites sur des patients théoriques dont la résistance du point zéro a été fixée à 250 kΩ, est mémorisée dans l'ordinateur, que l'ordinateur recalcule la mesure en se référant à des patients théoriques dont la résistance du point zéro est 250 kΩ.

2. Instrument de mesure de la valeur résistive de la peau aux points réflexologiques pour le secteur électromédical selon la revendication 1, **caractérisé par le fait que** le point de contact (8) de la sonde de mesure (6) est rétractable d'une position entièrement sortie à une position entièrement rentrée et assure une pression de 65 g entre le point de contact (8) et la peau (1).

3. Instrument de mesure de la valeur résistive sur la peau aux points réflexologiques pour le secteur électromédical selon la revendication 2, **caractérisé par le fait que** le point de contact (8) offre à la peau (1) une surface de contact (10) avec une forme sphérique abaissée de manière uniforme dont le diamètre maximal est de 1,5 mm tandis que le diamètre minimal est de 0,66 mm.

4. Instrument de mesure de la valeur résistive sur la peau aux points réflexologiques pour le secteur électromédical selon la revendication 2 ou 3, **caractérisé par le fait que** le point de contact (8) est fait d'une matière conductrice.

5. Méthode de mesure réflexologique qui utilise un instrument de mesure de la valeur résistive sur la peau aux points réflexologiques pour le secteur électromédical selon l'une quelconque des revendications précédentes, **caractérisée par le fait que**

   - le réseau des commutateurs analogiques placés sur la polarité négative, est programmé de telle façon qu'il peut être totalement activé, donnant ainsi à cette section une valeur de résistance égale à la valeur de la résistance du point zéro ;
   - le réseau des commutateurs de polarité analogiques (SPLRT) est programmé de telle façon que seuls les commutateurs analogiques $S_{WC2}$ et $S_{WC4}$ sont actifs ; afin de rendre le réseau de mesure opérationnel sur le pôle positif ;
   - le réseau des commutateurs analogiques placés sur la polarité négative, est programmé de telle façon qu'il peut être totalement activé et donner ainsi à cette section une valeur de résistance égale à la valeur de la résistance du point de l'oreille en cours d'examen.

6. Méthode de mesure réflexologique qui utilise un instrument de mesure de la valeur résistive sur la peau aux points réflexologiques pour le secteur électromédical selon la revendication 5, **caractérisée par le fait que** les commutateurs de polarité analogiques (SPLRT):

   a) ferment électriquement le point réflexologique en cours d'examen en activant des commandes : $S_{WC1}$, $S_{WC2}$, $S_{WC3}$, $S_{WC4}$ ;
   b) ouvrent électriquement le point réflexologique en cours d'examen en désactivant les commandes : $S_{WC1}$, $S_{WC2}$, $S_{WC3}$, $S_{WC4}$ ;
   c) polarisent d'une façon positive le point réflexologique en cours d'examen en activant des commandes : $S_{WC2}$,

$S_{WC4}$ ;
d) polarisent d'une façon négative le point réflexologique en cours d'examen en activant des commandes :
$S_{WC1}$, $S_{WC3}$.

fig. 1

fig. 2

fig. 5

fig. 3

fig. 4

fig. 6

fig. 7

fig. 8